(19) Europäisches Patentamt
European Patent Office
Office européen des brevets

(11) Veröffentlichungsnummer: **0 266 691 B1**

# (12) EUROPÄISCHE PATENTSCHRIFT

(45) Veröffentlichungstag der Patentschrift: **18.09.91**

(51) Int. Cl.5: **C07C 69/533**, C07C 67/327

(21) Anmeldenummer: **87115959.6**

(22) Anmeldetag: **30.10.87**

## (54) Verfahren zur Herstellung von Alkencarbonsäureestern.

(30) Priorität: **07.11.86 DE 3638010**

(43) Veröffentlichungstag der Anmeldung:
**11.05.88 Patentblatt 88/19**

(45) Bekanntmachung des Hinweises auf die Patenterteilung:
**18.09.91 Patentblatt 91/38**

(84) Benannte Vertragsstaaten:
**BE CH DE ES FR GB IT LI NL**

(56) Entgegenhaltungen:
**EP-A- 0 206 143**
**EP-A- 0 238 002**

**CHEMICAL ABSTRACTS, Band 82, Nr. 19, 1975, Seiten 505-506,Spalten 2,1, Abstract Nr. 124673j, Columbus, Ohio, USA; Y. MURAMOTO et al, "Alcoholysis of lactones in the presence of magnesium alkoxide. Preparation of 6-hydroxyhexanoates and 5-hydroxypentanoates"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Näumann, Fritz, Dr.**
**Rathenaustrasse 3 a**
**W-6800 Mannheim1(DE)**
Erfinder: **Fischer, Rolf, Dr.**
**Bergstrasse 98**
**W-6900 Heidelberg(DE)**

**EP 0 266 691 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Anmeldung betrifft ein Verfahren zur Herstellung von Alkencarbonsäureestern durch Umsetzung von Lactonen mit Alkoholen in Gegenwart von Katalysatoren.

Aus Nippon Nogei Kogaku Kaishi 1974, 48, Seiten 525 bis 527; C.A. 82, 124.673 (1975) ist bekannt, daß man omega-Hydroxycarbonsäureester durch Umsetzen von 5- bis 7-gliedrigen Lactonen mit Alkoholen in Gegenwart von Magnesiumalkoholaten oder Säuren erhält.

Auch ist es bekannt, daß 3-Pentensäureester bei Temperaturen von 100 bis 150° C in Gegenwart von sauren Ionenaustauschern oder sauren Zeolithen, die Edelmetalle der achten Gruppe des periodischen Systems wie Palladium, Rhodium oder Ruthenium enthalten, zu 4-Pentenestern zu isomerisieren (DE-OS 33 17 163). So erhält man z.B. ausgehend von 3-Pentensäuremethylester (70 % trans, 30 % cis) Isomerengemische, die neben unumgesetztem 3-Pentensäuremethylester und geringen Mengen 2-Pentenestern 8 Gew.-% des gewünschten 4-Pentensäuremethylesters enthalten. Der 4-Pentensäureester läßt sich aus derartigen Isomerengemischen durch eine Azeotropdestillation mit Wasser abtrennen (DE-OS 34 12 295). Nachteilig an dieser Vorgehenweise ist, daß sich der 4-Pentensäureestergehalt der Isomerengemische nicht wesentlich über 8 Gew.-% steigern läßt. Die Folge ist, daß für die Abtrennung des 4-Pentensäureesters und die Rückführung der 2- und 3-Pentensäureester ein beträchtlicher Destillationsaufwand entsteht.

Es war die technische Aufgabe gestellt, ein einstufiges Verfahren zur Herstellung von Alkencarbonsäureestern mit endständiger Doppelbindung ausgehend von entsprechenden 5- bis 7-gliedrigen Lactonen zur Verfügung zu stellen.

Weiterhin bestand die technische Aufgabe, ein Verfahren zu entwickeln, bei dem höhere 4-Pentensäureester-Gehalte im Pentensäureester-Isomerengemisch erzielt werden.

Diese Aufgabe wird gelöst in einem Verfahren zur Herstellung von Alkencarbonsäureestern der Formel I

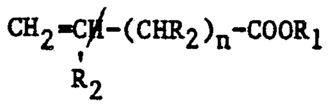

I,

in der $R^1$ einen Alkylrest mit 1 bis 6 Kohlenstoffatomen bezeichnet, $R^2$ für ein Wasserstoffatome oder für einen Alkylrest mit 1 bis 4 Kohlenstoffatomen steht, wobei die Substituenten gleich oder verschieden sein können und n für 1, 2 oder 3 steht, durch Umsetzen von Lactonen der Formel II

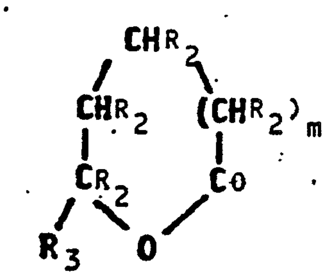

II,

in der $R^2$ die gleiche Bedeutung wie in Formel I hat, $R_3$ ein Wasserstoffatom oder eine Methylgruppe bezeichnet und m für 0 oder 1 oder 2 steht mit der Maßgabe, daß die Differenz von n und m für den Fall, daß $R_3$ Wasserstoff bedeutet, den Wert 1 und für den Fall, daß $R_3$ Methyl bedeutet, den Wert 2 hat,
mit Alkanolen mit 1 bis 6 Kohlenstoffatomen bei einer Temperatur von 50 bis 450° C in Gegenwart von Katalysatoren, wobei man Zeolithe und/oder Phosphate als Katalysatoren verwendet. Hierbei ist es besonders vorteilhaft, in der Gasphase bei einer Temperatur von 100 bis 450° C zu arbeiten.

Das neue Verfahren hat den Vorteil, daß man in einer Stufe, ausgehend von 5- bis 7-gliedrigen Lactonen in guten Ausbeuten zu Alkencarbonsäureestern mit endständiger Doppelbindung gelangt. Ferner hat das neue Verfahren den Vorteil, daß es technisch auf einfache Weise durchführbar ist und man bei der Verwendung von 5-Methylbutyrolacton bessere Ausbeuten an 4-Pentensäureestern erhält.

Das neue Verfahren ist insofern bemerkenswert, als die Lactonspaltung zu Alkencarbonsäureestern gegenüber der ebenfalls sauer katalysierten Etherbildung aus den beteiligten Alkoholen bevorzugt ist.

Auch ist die Bildung von Pentensäureestern und insbesondere 4-Pentensäureestern bei der Umsetzung von 5-Methylbutyrolacton mit Alkoholen in Gegenwart von Zeolithen und/oder Phosphaten als Katalysatoren überraschend und wurde bisher nicht beschrieben.

Es war weiterhin nicht vorauszusehen, daß bei der Spaltung von 5-Methylbutyrolacton mit Alkoholen in Gegenwart von Zeolithen und/oder Phosphaten als Katalysatoren neben cis- und trans-3-Pentensäureestern 4-Pentensäureester in hohen Ausbeuten gefunden wird und die Bildung von 2-Pentensäureestern stark zurückgedrängt bzw. gänzlich vermieden werden kann.

Dies ist insbesondere deshalb von großer Bedeutung, da beispielsweise 2-cis-Pentensäuremethylester aufgrund des gleichen Siedepunktes (129 °C) von 4-Pentensäuremethylester destillativ nicht getrennt werden kann.

Erfindungsgemäß geht man von Lactonen der Formel II aus. Geeignete Lactone sind beispielsweise Caprolacton, 7-Methylcaprolacton, delta-Valerolacton, 6-Methylvalerolacton, Butyrolacton oder 5-Methylbutyrolacton. Bevorzugte Ausgangsstoffe sind Caprolacton, delta-Valerolacton und 5-Methylbutyrolacton.

Die Umsetzung wird mit Alkanolen mit 1 bis 6 Kohlenstoffatomen durchgeführt. Geeignete Alkanole sind beispielsweise Methanol, Ethanol, Propanol, Butanol, Isopropanol oder Isobutanol, sekundäre Butanole, n-Pentanol oder n-Hexanol. Besonders geeignet sind Methanol, Ethanol und Propanole.

Die erfindungsgemäße Umsetzung läßt sich z.B. für den Fall der Herstellung von 4-Pentensäuremethylester durch die folgende Formelgleichung wiedergeben:

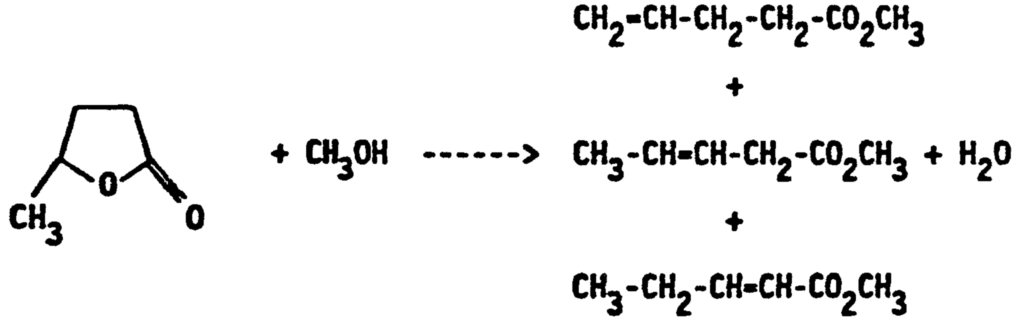

Neben 4-Pentensäuremethylester werden 3- und 2-Pentensäuremethylester gebildet, die mit Ausnahme von 2-cis-Pentensäuremethylester (der aber nur in geringer Menge anfällt), destillativ von 4-Pentensäuremethylester abgetrennt werden können.

Als Katalysatoren für das erfindungsgemäße Verfahren werden acide zeolithische Katalysatoren eingesetzt. Zeolithe sind z.B. kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2 (s. Ullmanns Encyclopädie d. techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf, ersetzt werden.

Entsprechend ihrer Struktur werden Zeolithe in verschiedene Gruppen unterteilt (s. Ullmanns Encycopädie d. techn. Chemie, 4. Aufl., Bd. 24, S. 575 (1983)). So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind vorzugsweise Zeolithe aus der Mordernit-Gruppe oder engporige Zeolithe vom Erionit- bzw. Chabasit-Type oder Zeolithe vom Faujasit-Typ, z.B. Y-, X- oder L-Zeolithe. In diese Gruppe von Zeolithen gehören auch die sogenannten "ultrastabile" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et al Elsevier Scientific Publishing Comp. 1980, S. 203 and "Crystal Structures of Ultra-stable Faujasites" Advances in Chemistry Series Nr. 101, American Chemical Society Washington, D.C. S. 226 ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeich-

net sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, Seite 575, 1983.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithode oder deren deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220° unter autogenem Druck hergestellt. Auch gehören hierzu die isotaktischen Zeolithe nach DE-OS 3 006 471. Die erhaltenen Aluminosilikatzeolithe enthalten je nach Wahl der Einsatzstoffmengen ein $SiO_2/Al_2O_3$-Verhältnis von 10 bis 40 000. Auch lassen sich derartige Aluminosilikatzeolithe in ehterischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200° C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach DE-OS 3 006 471 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugweise $Fe_2(SO_4)_3$ und einer Siliciumverbidung, vorzugsweise hochdispersem Siliciumdioxid in wäßriger Aminlösung, insbesondere 1,6-Hexandiamin, mit oder ohne Alkali- oder Erdalkalizusatz bei 100 bis 220° C unter autogenem Druck.

Zu den verwendbaren siliciumreichen Zeolithen ($SiO_2/Al_2O_3$ 10) gehören auch die sog. ZSM-Typen, z.B.

- ZSM 5 ist ein kristalliner Aluminosilikatzeolith, der in US-PS 3 702 886 offenbart ist. Die Herstellung ist beispielsweise in den Beispielen 1, 2, 6, 22, 26 und 27 von US-PS 3 702 886 beschrieben.
- ZSM 8 ist ein kristalliner Aluminosilikatzeolith, der in US-PS 3 709 979 geschützt ist. Seine Herstellung ist in den Beispielen 1, 2, 4, 5, 8 und 10 mitgeteilt.
- ZSM 12 ist ein kristalliner Aluminosilikatzeolith, der in US-PS 3 832 449 offenbart ist, und dessen Herstellung in den Beispielen 1 bis 8 ausgeführt ist.
- ZSM 21 ist ein kristalliner Zeolith, der in US-PS 4 046 859 offenbart ist.
- ZSM 22 ist ein kristalliner Zeolith, der in EP 102 716 offenbart ist.
- ZSM 23 ist ein kristalliner Zeolith, der in US-PS 4 076 842 offenbart ist.
- ZSM 25 ist ein kristalliner Zeolith, der in US-PS 4 247 416 beschrieben ist.
- ZSM 34 ist ein kristalliner Aluminosilikatzeolith, der in US-PS 4 086 186 offenbart ist.
- ZSM 35 ist ein kristalliner Zeolith, der in US-PS 4 016 245 und 4 107 195 offenbart ist.
- ZSM 48 ist ein kristalliner Zeolith, der in US-PS 4 448 675 offenbart ist.
- Ferrierit ist ein kristalliner Zeolith, der in EP 12 473 offenbart ist.
- NU-1 ist ein kristalliner Zeolith, der in US-PS 4 060 590 offenbart ist.
- Silicalite® ist ein Molekularsieb, ein sog. Silica Polymorph, welches detailliert in US-PS 4 061 724 offenbart ist.

Die so hergestellten Alumino-, Boro- und Eisensilikatzeolithe können nach ihrer Isolierung, Trocknung bei 100 bis 160° C, vorzugsweise 110° C und Calcinierung bei 450 bis 550° C, vorzugsweise 500° C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge z.B. bei 110° C/16 h getrocknet und bei 500° C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolith können in reiner Form, ohne Binder, als Stränge oder Tabletten eingesetzt werden, wobei als Verstrangungs-oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor,

sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Ir, Ni, Pd, Pt, Übergangsmetalle oder 1. und 2. Nebengruppe wie Cu, Ag, Zn, seltene Erdmetalle wie La, Ce, Pr, Nd, Fr, Yb und U eingesetzt. Der Gehalt an den genannten Metallen beträgt vorteilhaft 0,1 bis 3 Gew.-%, berechnet als Metall.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20h leitet. Danach wird mit Wasser ausgewaschen, bei ca. 150 °C getrocknet und bei ca. 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe - vor oder nach ihrer Verformung mit Bindemitteln - z.B. 1 bis 3 Stunden bei Temperaturen nach 60 bis 80 °C mit einer 3 bis 25 Gew.-%igen, insbesondere 12 bis 20 Gew.-%igen wäßrigen Salzsäure.

Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 9,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials wird dieses zweckmäßig, z.B. bei

Temperaturen von 100 bis 160° C, getrocknet und bei Temperaturen von im allgemeinen 450° C bis 600° C calciniert. Gemäß einer weiteren bevorzugten Ausführungsform für die Säurebehandlung wird das zeolithische Material nach einer Verformung mit Bindemitteln bei erhöhter Temperatur, zweckmäßig bei Temperaturen von 50° C bis 90° C, vorzugsweise 60° C bis 80° C, über einen Zeitraum von 0,5 bis 5, vorzugsweise mit 12 bis 20 Gew.-%iger Salzsäure, behandelt. Anschließend wird das zeolithische Material im allgemeinen ausgewaschen und zweckmäßig, z.B. bei Temperaturen von 100 bis 160° C, getrocknet und bei Temperaturen von im allgemeinen 450 bis 600° C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten-oder Wirbelgut-Form mit wäßriger $NaH_2PO_4$-Lösung getränkt, bei 110° C getrocknet und bei 500° C calciniert.

Weitere Katalysatoren für die Herstellung von Alkencarbonsäureestern aus Lactonen sind Phosphate, insbesondere Aluminiumphosphate, Siliciumaluminiumphosphate,Siliciumeisenaluminiumphosphate, Eisenaluminiumphosphat, Cerphosphat, Zirkonphosphate, Borphosphat, Eisenphosphat oder deren Gemische. Vorteilhaft sind hydrothermal hergestellte Phosphate.

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate sind z.B. APO-5, APO-9, APO-12, APO-14, APO-21, APO-25, APO-31 und APO-33. Synthesen dieser Verbindungen sind in EP 132 708, US 4 310 440 und US 4 473 663 beschrieben.

Beispielsweise wird das $AlPO_4$-5 (APO-5) synthetisiert, indem man Orthophosphorsäure mit Pseudoboehmit in Wasser homogen mischt, zu dieser Mischung Tetrapropylammoniumhydroxid gibt und danach bei ca. 150° C 20 bis 60 h unter autogenem Druck in einem Autoklaven umsetzt. Das abfiltrierte $AlPO_4$ wird getrocknet bei 100 bis 160° C und calciniert bei 450 bis 550° C.

$AlPO_4$-9 (APO-9) wird ebenfalls aus Orthophosphorsäure und Pseudoboehmit, aber in wäßriger DABCO-Lösung (1,4-Diazabicyclo-(2,2,2)-octan) bei ca. 200° C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidin-Lösung bei 150 bis 200° C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250° C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 wird beispielsweise durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200° C während 20 bis 200 h unter autogenem Druck in einem Rührautoklaven erhalten. Die Trocknung des abfiltrierten Puvlers erfolgt bei 110 bis 160° C und die Calcination bei 450 bis 550° C.

Si-$AlPO_4$-11 (SAPO-11) wird z.B. synthetisiert aus einer Mischung von 200 g $H_3PO_4$, 136 g AlOOH, 60 g Kieselsol (30%ig), 91 g Dipropylamin und 890 g Wasser. Die Umsetzung wird bei 200° C während 96 Stunden unter autogenem Druck durchgeführt. Nach Filtrieren wird bei 120° C getrocknet und bei 500° C calciniert.

Als Siliciumaluminiumphosphate sind z.B. ZYT-5, ZYT-6, ZYT-7, ZYT-9, ZYT1-11 und ZYT-12 geeignet (J 5 9217-619).

Als Phosphat-Katalysatoren werden für das erfindungsgemäße Verfahren auch gefällte Aluminiumphosphate eingesetzt. Beispielsweise wird ein derartiges Aluminiumphosphat hergestellt, indem 92 g Diammoniumhydrogenphosphat in 700 ml Wasser gelöst werden, zu dieser Lösung 260 g $Al(NO_3)_3$ x $H_2O$ in 700 ml Wasser über 2 h zugegeben wird, wobei der pH-Wert durch gleichzeitige Zugabe von 25%iger $NH_3$-Lösung bei pH 8 gehalten wird. Der entstandene Niederschlag wird 12 h nachgerührt, dann abgesaugt und ausgewaschen. Er wird bei 60° C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650° C, vorzugsweise 300 bis 500° C herstellen.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt

eingesetzt werden.

Für das erfindungsgemäße Verfahren werden im allgemeinen folgende Reaktionsbedingungen gewählt: Das Molverhältnis von Lactonen der Formel II zu Alkanolen beträgt vorteilhaft von 1 : 0,5 bis 1 : 10, insbesondere 1 : 1 bis 5.

Bei der Umsetzung hält man eine Temperatur von 50 bis 450° C ein. Die Umsetzung führt man insbesondere in der Gasphase bei einer Temperatur von 100 bis 450° C durch. Vorteilhaft hält man eine Temperatur von 150 bis 400° C, insbesondere 200 bis 350° C ein. In der Regel führt man die Umsetzung bei einem Druck von 0,1 bis 100 bar, insbesondere 0,5 bis 10 bar durch.

Setzt man Lactone der Formel II an oben beschriebenen Katalysatoren in der Gasphase um, so hält man vorteilhaft eine Katalysatorbelastung von 0,1 bis 20, insbesondere von 1 bis 10 g Lacton der Formel II je g Katalysator und Stunde ein (WHSV = g Einsatzgemisch/g Katalysator und Stunde).

Die Reaktionen in der Gasphase können in einem Festbett oder in einem Wirbelbett ausgeführt werden.

Auch ist es möglich, die Reaktion in der Flüssigphase, z.B. in Suspension-, Riesel- oder Sumpffahrweise, bei Temperaturen zwischen 50 und 200° C durchzuführen.

Die Reaktion kann diskontinuierlich durchgeführt werden, es wird aber bevorzugt, sie kontinuierlich ablaufen zu lassen.

Schwerflüchtige oder feste Ausgangsstoffe werden in gelöster Form, z.B. in Tetrahydrofuran-, Toluol- oder Petrolether-Lösung zum Einsatz gebracht. Generell ist eine Verdünnung der Ausgangsstoffe mit derartigen Lösungsmitteln oder mit Inertgasen wie $N_2$, Ar, $H_2O$-Dampf möglich.

Nach der Umsetzung werden die entstandenen Produkte durch übliche Techniken, z.B. durch Destillation aus dem Reaktionsgemisch isoliert; nichtumgesetztes Einsatzgemisch wird gegebenenfalls, wenn nötig, für die erfindungsgemäße Umsetzung zurückgeführt.

Die nach dem erfindungsgemäßen Verfahren erhältlichen omega-Alkencarbonsäureester sind vielseitig verwendbare Zwischenprodukte, z.B. zur Herstellung von alpha-, omega-Dicarbonsäuren.

Das erfindungsgemäße Verfahren eignet sich besonders zur Herstellung von 4-Pentensäureestern und besitzt gegenüber der Isomerisierung von 3-Pentenestern zu 4-Pentenestern der Vorteil, daß höhere 4-Pentenestergehalte im Pentenester-Isomerengemisch erreicht werden. Hierdurch gestaltet sich die Abtrennung der 4-Pentenester günstiger. 2- und 3-Pentensäureester lassen sich nach Verseifung zu den entsprechenden Pentensäuren in Gegenwart von Schwefelsäure als Katalysator wieder in 5-Methylbutyrolacton verwandeln (R.P. Linstead, J. Chem. Soc. 1932, Seiten 115 bis 129).

Die nach dem erfindungsgemäßen Verfahren erhältlichen 4-Pentensäureester stellen wertvolle Zwischenprodukte dar, die sich durch Niederdruck-Hydroformylierung in Gegenwart von Rh-Verbindungen als Katalysatoren in 5-Formylvaleriansäureester umwandeln lassen (DE-OS 33 17 164). 5-Formylvaleriansäureester können durch aminierende Hydrierung und Cyclisierung der entstehenden Aminocapronester ohne Ammonsulfat-Anfall zu Caprolactam umgesetzt werden.

Das Verfahren nach der Erfindung sei an folgenden Beispielen veranschaulicht.

Beispiele 1 bis 13

5-Methylbutyrolacton und Methanol im Molverhältnis 1 : 3 wurden verdampft und in der Gasphase unter isothermen Bedingungen in einem Rohrreaktor (Wendel, 0,6 cm Innendurchmesser, 90 cm Länge), der mit dem Katalysator beschickt ist, im Verlauf von 6 Stunden umgesetzt. Die Reaktionsprodukte wurden kondensiert abgetrennt und charakterisiert. Die quantitative Bestimmung der Reaktionsprodukte und der Ausgangsprodukte erfolgte gaschromatographisch. Die angewandten Temperaturen, Katalysatoren und Mengenverhältnisse sowie die erzielten Ergebnisse sind aus Tabelle 1 ersichtlich.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 : 50 Gew.-%) bei 170° C unter autogenem Druck in einem Rührautoklaven hergestellt. Nach Abfiltrieren und Auswaschen wird das kristalline Reaktionsprodukt bei 100° C/24 h getrocknet und bei 500° C/2 h calciniert. Dieser Borosilikatzeolith setzt sich zusammen aus 94,2 Gew.-% $SiO_2$ und 2,3 Gew.-% $B_2O_3$.

Mit diesem Material werden durch Verformen mit einem Verformungshilfsmittel 2-mm-Stränge hergestellt, die bei 110° C/16 h getrocknet und bei 500° C/24 h calciniert werden.

Katalysator B

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50 : 50 Gew.-%) in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminosilikatzeolith enthielt 91,6 Gew.-% $SiO_2$ und 4,6 Gew.-% $Al_2O_3$. Der Katalysator wurde zu 2-mm-Strängen verformt, bei 110 °C/16 h getrocknet und bei 500 °C/24 h calciniert.

Katalysator C

Kommerziell erhältlicher Na-Y-Zeolith wird mit wäßriger $(NH_4)_2SO_4$-Lösung nach bekannter Methode ionenausgetauscht, so lange bis der Na-Gehalt kleiner 0,05 Gew.-% liegt (nach Trocknung 110 °C/2 h und Calcination bei 570 °C/3 h). Das so erhaltene Pulver wird mit Verformungshilfsmitteln zu Strängen verformt, bei 110 °C getrocknet und bei 500 °C/16 h calcinert.

Katalysator D

$AlPO_4$-9 (APO-9) wird synthetisiert, indem man 200 g 98%ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200 °C während 336 Stunden unter autogenem Druck umsetzt. Nach Abfiltrieren wird das kristalline Material bei 120 °C getrocknet und 500 °C/16 h calciniert. Das so synthetisierte $AlPO_4$-9 enthält 49,0 Gew.-% $P_2O_5$, 37,1 Gew.-% $Al_2O_3$. Dieses Material wird mit hochdispersem $SiO_2$ im Gewichtsverhältnis 80 : 20 zu 3-mm-Strängen verformt, abermals bei 120 °C getrocknet und bei 500 °C/6 h calciniert.

Katalysator E

$SiAlPO_4$-5 (SAPO-5) wird hergestellt aus einer Mischung aus 200 g 98%ige Phosphorsäure, 136 g Boehmit, 60 g Kieselsol (30%ig), 287 g Tripropylamin und 587 g $H_2O$. Diese Mischung wird bei 150 °C während 168 Stunden unter autogenem Druck umgesetzt. Nach Filtration wird das kristalline Produkt bei 120 °C getrocknet und bei 500 °C calciniert. SAPO-5 enthält 49,8 Gew.-% $P_2O_5$, 33,0 Gew.-% $Al_2O_3$, 6,2 Gew.-% $SiO_2$. SAPO-5 wird mit einem Verstrangungsmittel zu 3-mm-Strängen verformt, bei 120 °C getrocknet und bei 500 °C calciniert.

Katalysator F

Katalysator F wird erhalten, indem man Katalysator A mit 20%iger wäßriger $LiNO_3$-Lösung bei 80 °C/2 h ionenaustauscht. Danach wird mit $H_2O$ ausgewaschen, bei 110 °C getrocknet und bei 500 °C/5 h calciniert. Der Li-Gehalt beträgt 0,24 Gew.-%.

Katalysator G

$BPO_4$ wird hergestellt, indem man 49 g $H_3BO_3$ mit 117 g $H_3PO_4$ (75%ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator G enthält 8,77 Gew.-% B und 28,3 Gew.-% P.

Katalysator H

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator H enthält 47,1 Gew.-% Ce und 12,7 Gew.-% P.

Katalysator I

Im Handel erhältliches $Zr_3(PO_4)_4$ wird mit Verformungshilfsmitteln zu 2-mm-Strängen verformt, bei 110 °C getrocknet und bei 500 °C/16 h calciniert.

Katalysator J (Vergleichskatalysator)

$Al_2O_3$-Katalysator

Katalysator K (Vergleichskatalysator)
$SiO_2$-Katalysator

Die Versuchsergebnisse sind in Tabelle 1 zusammengestellt. Angaben über Reaktionsbedingungen und die erzielten Ergebnisse sind dort aufgeführt.

Die Vergleichsbeispiel mit den Vergleichskatalysatoren K und L zeigen, daß die Umsätze und Selektivitäten für 3- und 4-PSE in Summe niedriger liegen als beim erfindungsgemäßen Verfahren, und daß mehr 2-Pentensäuremethylester gebildet werden (Differenz Summe aller PSE - 4-PSE - 3-PSE).

Beispiel 14

5-Methylbutyrolacton wird mit $CH_3OH$ (1 : 3 Molverhältnis) am Katalysator A bei 220°C und WHSV = 1,6 $h^{-1}$ umgesetzt. Während einer Laufzeit von 160 h zeigt der 4-Pentensäureestergehalt (4-PSE) keine nennenswerte Veränderung (Tabelle 2).

Tabelle 2

| Zeit h | 6 h | 46 h | 94 h | 138 h | 154 h |
|---|---|---|---|---|---|
| 4-PSE Fl.-% | 19,9 | 19,8 | 20,2 | 19,4 | 20,8 |

Beispiel 15

Ein Gemisch aus delta-Valerolacton und Methanol (1 : 3 Molverhältnis) wird in der voranbeschriebenen Apparatur bei 300°C und WHSV = 1,5 $h^{-1}$ am Katalysator A umgesetzt. Der Umsatz beträgt 99,1 %. Hierbei werden Selektivitäten von 4-PSE mit 12 %, von 3-PSE mit 48,0 %, von 2-PSE mit 19,5% und von 5-Methylbutyrolacton von 14,4 % erzielt. Da 5-Methylbutyrolacton ebenfalls in Pentensäureester (PSE) überführt werden kann, läßt sich die Gesamtselektivität von PSE auf ca. 94 % steigern.

Beispiel 16

Ein Gemisch aus Caprolacton und Methanol (1 : 3 Molverhältnis) wird in der voranbeschriebenen Apparatur bei 300°C und WHSV = 1,8 $h^{-1}$ an Katalysator A umgesetzt. Der Umsatz beträgt 98,6 % und die Selektivität der Hexensäuremethylester 88,8 %.

Beispiel 17

Ein in einem Salzbad thermostatisierter Reaktor (Durchmesser innen 2,4 cm. Länge 49 cm, V4A) wurde mit 50 g Katalysator A beschickt. Unter isothermen Bedingungen wird ein Gemisch aus Caprolacton und Methanol (Molverhältnis 1:3) umgesetzt. Die Temperatur wurde im Bereich 300 und 350°C variiert. Die Belastung betrug 41 g des Gemisches pro Stunde. Weiterhin wurden 30 l $N_2$/h als Purgegas zugeleitet. Die Ausbeute an Hexensäuremethylester lag während einer 14-tägigen Fahrperiode zwischen 80 und 787 %.

Tabelle 1

Reaktion von 5-Methylbutyrolacton mit Methanol (1 : 3 molar) zu Pentensäuremethylester

| Beispiel | 1 | 2 | 3 | 4 | 5 | 6 | 7 | 8 | 9 | 10 | 11* | 12* |
|---|---|---|---|---|---|---|---|---|---|---|---|---|
| Katalysator | A | A | B | C | D | E | F | G | H | I | J* | K* |
| Temperatur | 220°C | 300°C | 300°C | 250°C | 300°C | 300°C | 300°C | 300°C | 300°C | 300°C | 220°C | 300°C |
| WHSV [$h^{-1}$] | 1,5 | 10 | 6 | 1,5 | 1,5 | 1,5 | 1,5 | 1,5 | 1,5 | 1,5 | 1,5 | 1,5 |
| Umsatz % | 65,1 | 66,9 | 67,0 | 60,6 | 72,2 | 59,1 | 13,0 | 29,4 | 30,2 | 29,5 | 16,0 | 35,4 |
| Selektivität % | | | | | | | | | | | | |
| 4-PSE | 29,2 | 33,4 | 16,5 | 24,7 | 29,2 | 35,0 | 47,6 | 38,3 | 32,5 | 34,2 | 22,3 | 35,5 |
| 3-PSE | 67,0 | 61,4 | 71,9 | 69,6 | 40,9 | 38,1 | 21,5 | 38,4 | 41,1 | 37,3 | 21,9 | 44,1 |
| PSE | 96,9 | 97,8 | 98,1 | 91,7 | 98,5 | 98,6 | 85,7 | 95,9 | 96,9 | 94,9 | 59,0 | 93,5 |

PSE = Pentensäuremethylester

* Vergleichsbeispiele mit Vergleichskatalysatoren

## Patentansprüche

1. Verfahren zur Herstellung von Alkencarbonsäureestern der Formel I

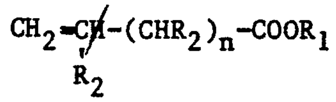

I,

in der $R_1$ einen Alkylrest mit 1 bis 6 Kohlenstoffatomen bezeichnet, $R_2$ für ein Wasserstoffatom oder für einen Alkylrest mit 1 bis 4 Kohlenstoffatomen steht, wobei die Substituenten gleich oder verschieden sein können, und n für 1, 2 oder 3 steht, durch Umsetzen von Lactonen der Formel II

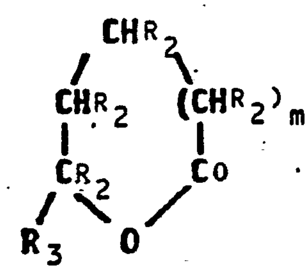

II,

in der $R^2$ die gleiche Bedeutung wie in Formel I hat, $R_3$ ein Wasserstoffatom oder eine Methylgruppe bezeichnet und m für 0, 1 oder 2 steht, mit der Maßgabe, daß die Differenz von n und m für den Fall, daß $R_3$ Wasserstoff bedeutet, den Wert 1 und für den Fall, daß $R_3$ Methyl bedeutet, den Wert 2 hat, mit Alkanolen mit 1 bis 6 Kohlenstoffatomen bei einer Temperatur von 50 bis 450 °C in Gegenwart von Katalysatoren, dadurch gekennzeichnet, daß man die Umsetzung in Gegenwart von Zeolithen und/oder Phosphaten als Katalysatoren durchführt.

2. Verfahren nach Anspruch 1, dadurch gekennzeichnet, daß man als Lacton der Formel II Caprolacton verwendet.

3. Verfahren nach Anspruch 1, dadurch gekennzeichnet, daß man als Lacton der Formel II delta-Valerolacton oder 6-Methylvalerolacton verwendet.

4. Verfahren nach Anspruch 1, dadurch gekennzeichnet, daß man als Lacton der Formel II Butyrolacton oder 5-Methylbutyrolacton verwendet.

5. Verfahren nach den Ansprüchen 1 bis 4, dadurch gekennzeichnet, daß man als Katalysator Zeolithe des Pentasiltyps verwendet.

6. Verfahren nach den Ansprüchen 1 bis 5, dadurch gekennzeichnet, daß man als Katalysatoren Bor-, Eisen- oder Aluminiumsilikatzeolithe des Pentasiltyps verwendet.

7. Verfahren nach den Ansprüchen 1 bis 4, dadurch gekennzeichnet, daß man als Katalysatoren Aluminiumsilikatzeolithe des Faujasit-Typs verwendet.

8. Verfahren nach den Ansprüchen 1 bis 7, dadurch gekennzeichnet, daß man als Katalysatoren mit Alkalimetallen oder mit Erdalkalimetallen oder mit Übergangsmetallen oder mit Seltenen Erden dotierte Zeolithe verwendet.

9. Verfahren nach den Ansprüchen 1 bis 4, dadurch gekennzeichnet, daß man als Katalysatoren Phosphate der Elemente Bor, Aluminium, Cer, Zirkonium, Eisen, Strontium oder deren Gemische verwendet.

10. Verfahren nach den Ansprüchen 1 bis 4 und 9, dadurch gekennzeichnet, daß man als Katalysatoren hydrothermal hergestellte Phosphate verwendet.

11. Verfahren nach den Ansprüchen 1 bis 4, 9 und 10, dadurch gekennzeichnet, daß man als Katalysatoren hydrothermal hergestellte Aluminiumphosphate oder Siliciumaluminiumphosphate oder Siliciumeisenaluminiumphosphate oder Eisenaluminiumphosphate verwendet.

12. Verfahren nach den Ansprüchen 1 bis 11, dadurch gekennzeichnet, daß man ein Molverhältnis von Lactonen der Formel II zu Alkanolen von 1 : 0,5 bis 1 : 10 einhält.

13. Verfahren nach den Ansprüchen 1 bis 12, dadurch gekennzeichnet, daß man die Umsetzung in der Gasphase durchführt.

## Claims

1. A process for preparing an alkenecarboxylic ester of the formula I

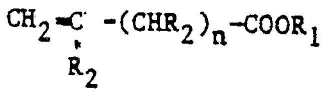

I

where $R_1$ is alkyl of 1 to 6 carbon atoms, $R_2$ is hydrogen or alkyl of 1 to 4 carbon atoms, the substituents being identical or different, and n is 1, 2 or 3, by reacting a lactone of the formula II

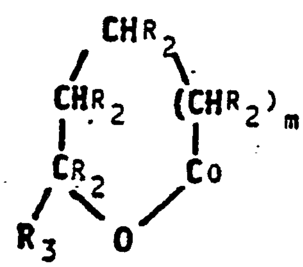

II

where $R^2$ has the same meanings as in the formula I, $R_3$ is hydrogen or methyl and m is 0, 1 or 2, with proviso that n and m differ by 1 when $R_3$ is hydrogen and by 2 when $R_3$ is methyl, with an alkanol of 1 to 6 carbon atoms at from 50 to 450 °C in the presence of a catalyst, which comprises carrying out the reaction in the presence of a zeolite or a phosphate as catalyst.

2. A process as claimed in claim 1, wherein the lactone of the formula II is caprolactone.

3. A process as claimed in claim 1, wherein the lactone of the formula II is delta-valerolactone or 6-methylvalerolactone.

4. A process as claimed in claim 1, wherein the lactone of the formula II is butyrolactone or 5-methylbutyrolactone.

5. A process as claimed in claim 1 or 2 or 3 or 4, wherein the catalyst used is a zeolite having a pentasil structure.

6. A process as claimed in claim 1 or 2 or 3 or 4 or 5, wherein the catalyst used is a borosilicate, iron silicate or aluminosilicate zeolite having a pentasil structure.

7. A process as claimed in claim 1 or 2 or 3 or 4, wherein the catalyst used is an aluminosilicate zeolite having a faujasite structure.

8. A process as claimed in claim 1 or 2 or 3 or 4 or 5 or 6 or 7, wherein the catalyst used is a zeolite doped with an alkali metal, an alkaline earth metal, a transition metal or a rare earth.

9. A process as claimed in claim 1 or 2 or 3 or 4, wherein the catalyst used is a phosphate of the element boron, aluminum, cerium, zirconium, iron or strontium or a mixture thereof.

**10.** A process as claimed in claim 1 or 2 or 3 or 4 or 9, wherein the catalyst used is a hydrothermally synthesized phosphate.

**11.** A process as claimed in claim 1 or 2 or 3 or 4 or 9 or 10, wherein the catalyst used is a hydrothermally synthesized aluminum phosphate, silicon aluminum phosphate, silicon iron aluminum phosphate or iron aluminum phosphate.

**12.** A process as claimed in claim 1 or 2 or 3 or 4 or 5 or 6 or 7 or 8 or 9 or 10 or 11, wherein a molar ratio of lactone of the formula II : alkanol of from 1 : 0.5 to 1 : 10 is maintained.

**13.** A process as claimed in claim 1 or 2 or 3 or 4 or 5 or 6 or 7 or 8 or 9 or 10 or 11 or 12, wherein the reaction is carried out in the gas phase.

**Revendications**

**1.** Procédé de préparation d'esters d'acides alcènecarboxyliques de formule I

$$CH_2{=}\underset{R_2}{\underset{|}{C}}-(CHR_2)_n-COOR_1 \qquad \text{I}$$

dans laquelle $R_1$ représente un reste alkyle à 1-6 atomes de carbone, $R_2$ est mis pour un atome d'hydrogène ou pour un reste alkyle à 1-4 atomes de carbone, les substituants pouvant être identiques ou différents, et n est mis pour 1, 2 ou 3, par réaction de lactones de formule II

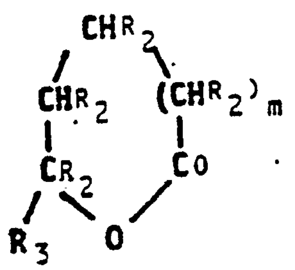

II,

dans laquelle $R_2$ a la même signification que dans la formule I, $R_3$ représente un atome d'hydrogène ou un groupement méthyle et m est mis pour 0, 1 ou 2, étant spécifié que la différence de n et m a la valeur 1 dans le cas où $R_3$ représente un atome d'hydrogène et la valeur 2 dans le cas où $R_3$ représente un groupement méthyle,
avec des alcanols à 1-6 atomes de carbone à une température de 50 à 450 °C en présence de catalyseurs, caractérisé en ce qu'on conduit la réaction en présence de zéolithes et/ou de phosphates en tant que catalyseurs.

**2.** Procédé selon la revendication 1, caractérisé en ce qu'on utilise de la caprolactone comme lactone de formule II.

**3.** Procédé selon la revendcation 1, caractérisé en ce qu'on utilise de la δ-valérolactone ou de la 6-méthylvalérolactone comme lactone de formule II.

**4.** Procédé selon la revendication 1, caractérisé en ce qu'on utilise de la butyrolactone ou de la 5-méthylbutyrolactone comme lactone de formule II.

**5.** Procédé selon l'une quelconque des revendications 1 à 4, caractérisé en ce qu'on utilise des zéolithes du type pentasil comme catalyseurs.

**6.** Procédé selon l'une quelconque des revendications 1 à 5, caractérisé en ce qu'on utilise des zéolithes

de boro-, ferro- ou aluminosilicate du type pentasil comme catalyseurs.

7. Procédé selon l'une quelconque des revendications 1 à 4, caractérisé en ce qu'on utilise des zéolithes d'aluminosilicate du type faujasite comme catalyseurs.

8. Procédé selon l'une quelconque des revendications 1 à 7, caractérisé en ce qu'on utilise, comme catalyseurs, des zéolithes dopées avec des métaux alcalins, avec des métaux alcalino-terreux, avec des métaux de transition ou avec des terres rares.

9. Procédé selon l'une quelconque des revendications 1 à 4, caractérisé en ce qu'on utilise, comme catalyseurs, des phosphates des éléments bore, aluminium, cérium, zirconium, fer, strontium ou de leurs mélanges.

10. Procédé selon l'une quelconque des revendications 1 à 4 et 9, caractérisé en ce qu'on utilise, comme catalyseurs, des phosphates préparés par voie hydrothermale.

11. Procédé selon l'une quelconque des revendications 1 à 4, 9 et 10, caractérisé en ce qu'on utilise, comme catalyseurs, des phosphates d'aluminium, des phosphates de silicium-aluminium, des phosphates de silicium-fer-aluminium ou des phosphates de feraluminium préparés par voie hydrothermale.

12. Procédé selon l'une quelconque des revendications 1 à 11, caractérisé en ce qu'on respecte un rapport molaire des lactones de formule II aux alcanols de 1:0,5 à 1:10.

13. Procédé selon l'une quelconque des revendications 1 à 12, caractérisé en ce qu'on conduit la réaction en phase gazeuse.